# EUROPEAN PATENT SPECIFICATION

(11) **EP 2 004 334 B1**
(45) Date of publication and mention of the grant of the patent: **14.11.2018**
(21) Application number: 07754937.6
(22) Date of filing: 04.04.2007
(51) Int. Cl.: B05B 17/06, B05B 12/02, B05B 12/12, A61L 9/14, B05B 12/00, A01M 1/20

(54) **ELECTRONIC AEROSOL DEVICE**
ELEKTRONISCHE AEROSOLVORRICHTUNG
DISPOSITIF AÉROSOL ÉLECTRONIQUE

(30) Priority: 11.04.2006 US 401572
(43) Date of publication of application: 24.12.2008
(73) Proprietor: S.C. Johnson & Son, Inc., Racine, WI 53403 (US)
(72) Inventor: HELF, Thomas, A., New Berlin, Wisconsin 53151 (US); FURNER, Paul, E., Racine, Wisconsin 53406 (US); PAAS, Edward, L., Los Altos, California 94022 (US)
(74) Representative: Ruschke, Hans Edvard
(86) International application number: PCT/US2007/008502
(87) International publication number: WO 2007/120565

(56) References cited:
- EP-A1- 0 258 637
- EP-A1- 1 184 083
- WO-A-97/31721
- WO-A-03/057291
- WO-A-03/068413
- WO-A-03/098971
- US-A- 4 702 418
- US-A- 5 452 711
- US-A1- 2005 169 812

## Description

### BACKGROUND OF THE INVENTION

### 1. Field of the Invention

The present disclosure relates to discharging a fluid from a spray device; and more particularly, to a method and apparatus for discharging a fluid through a nozzle using a piezoelectric pump assembly.

### 2. Description of the Background of the Invention

Manually-operated hand-held spray devices comprise pump-type sprayers that require repeated manual activation of a pump assembly to emit a fluid. Such spray devices are of limited usefulness because single action continuous spraying of a fluid cannot be accomplished. Instead, a user must repeatedly pump the assembly in order to emit a substantial quantity of product In other hand-held spray devices, such as aerosol containers, single action continuous spraying is achieved by opening a valve assembly to allow a pressurized propellant to emit a fluid stored within the container. However, aerosol containers utilize propellants to achieve this continuous spraying functionality. While such devices are more useful in those occasions when a substantial quantity of product is to be released, some consumers find the force necessary to hold the valve assembly in an open condition to result in hand fatigue. Also, the need for propellants and/or devices (such as a piston to contain the propellant in an application where the propellant is to remain isolated from the atmosphere) undesirably adds to the complexity and cost of the device.

US4702418 discloses an aerosol dispenser having a nozzle chamber having a nozzle, a reservoir and a piezoelectric bender to drive fluid from the reservoir to the nozzle. EP1184083 discloses a fragrance dispensing device having a reservoir and an electronically controlled liquid spray dispenser. WO03/098971 discloses a system having a fragrance dispenser, light source and audio system which are electronically controlled to achieve a desired ambiance.

### SUMMARY OF THE INVENTION

The invention is as set out in claim 1. Optional features are as set out in the dependent claims.

According to one embodiment of the present invention, a device for dispensing a fluid comprises a housing having an internal power source and a mounting assembly adapted for receiving a replaceable fluid reservoir. The fluid reservoir includes a capillary element for movement of the fluid to a discharge end thereof. A mechanism is disposed within the housing and is energized by the internal power source for vibrating a perforated discharge plate disposed adjacent the discharge end of the capillary element. The mechanism provides sufficient vibratory movement in a dispensing state to pump the fluid from the discharge end through the discharge plate and into the atmosphere. A control is carried by the housing and is disposed beneath the mounting assembly. The control provides an interface for a user to select at least one of a timed mode of operation, an automatic mode of operation dependent upon a sensor output developed by a sensor, and a manual mode of operation. The mounting assembly is further adapted to receive the replaceable fluid reservoir in a manner that allows same to be visually inspected during an in-use condition.

According to another embodiment of the present invention, a volatile liquid spraying device comprises a housing having an internal power source and a mounting assembly for receiving a replaceable fluid reservoir for holding a fluid. The fluid reservoir includes a capillary element for movement of the fluid to a discharge end thereof. A piezoelectric element is disposed within the housing and is energized by the internal power source for vibrating a perforated discharge plate disposed adjacent the discharge end of the capillary element. The piezoelectric element provides sufficient vibratory movement in a dispensing state to pump the fluid through the discharge plate and into the atmosphere. A control panel is disposed on the housing having an instant activation button and a switch for permitting the selection of a timed mode of operation and a sensor-based mode of operation for automatically operating the mechanism in response to a sensed parameter.

Also described is a hand-held spraying device comprising
a housing having a body, a bottom end, and a top end. A first chassis is slidingly retained within the body and movable between first and second positions. The housing is adapted to receive a battery therein. At least one activation device is disposed on the housing, A second chassis is disposed within the housing and retains a piezoelectric actuator and orifice plate assembly. The second chassis is further adapted to retain a removable liquid reservoir having a discharge end. The piezoelectric actuator and the orifice plate assembly are adapted to provide sufficient vibratory movement in a dispensing state to pump the liquid from the discharge end through the orifice plate. Movement of the first chassis to the first position allows the device to be placed in an operational state and movement of the first chassis into the second position allows at least one of the liquid reservoir and the battery to be inserted into the housing.

In a further embodiment of the present invention, a method for dispensing a fluid from a dispenser includes the step of providing a housing having an internal power source, a mounting assembly for receiving a replaceable fluid reservoir, a mechanism for vibrating a perforated discharge plate, and a control panel for activating the dispenser. The replaceable fluid reservoir is retained within the mounting assembly and the fluid reservoir includes a capillary element for movement of a fluid to a discharge end thereof. A fluid is provided within the replaceable fluid reservoir. The method further includes the steps of orienting the replaceable fluid reservoir within the mounting assembly.to allow a user to determine a level of the fluid within the fluid reservoir and activating the mechanism in at least one of a timed mode of operation, an automatic mode of operation dependent upon an output of a sensor, and a manual mode of operation. Activation causes the perforated discharge plate to vibrate adjacent the discharge end of the capillary element and pump the fluid from the discharge plate and into the atmosphere. The method includes the further step of energizing the mechanism by the internal power source.

Other aspects and advantages will become apparent upon consideration of the following detailed description and the attached drawings, in which like elements are assigned like reference numerals.
second elongate portions 38, 40 within the top portion 12. The rear side 34 also includes a mounting orifice 44 for attaching the housing 4 to a vertical or other support surface (not shown).

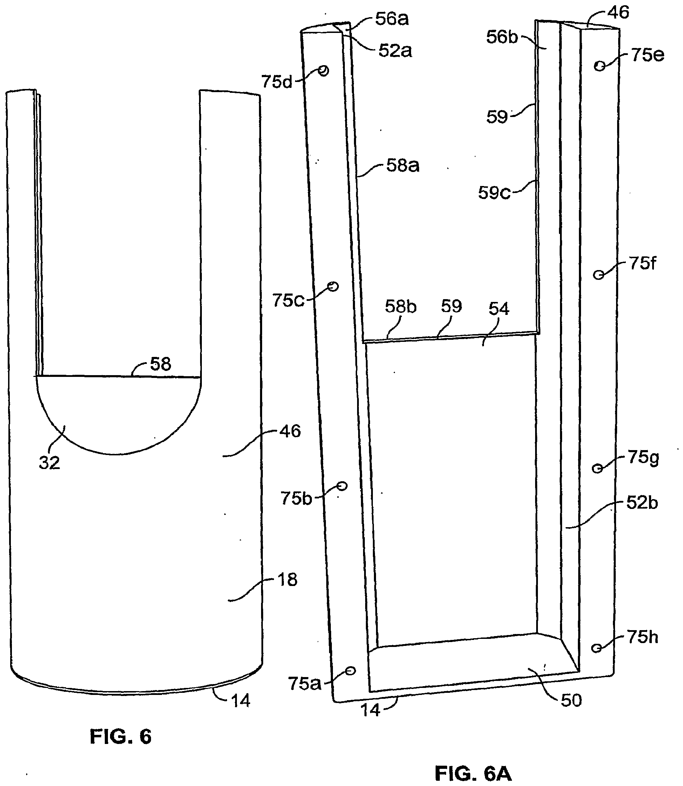

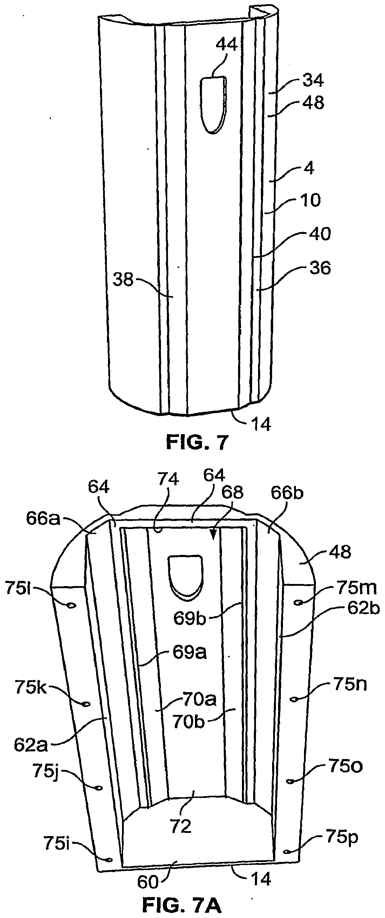

The housing 4 further comprises first and second body portions 46, 48, respectively. The first body portion 46, as shown in FIGS. 6 and 6A, forms the front side 18 of the cylindrical body 10 and a portion of the bottom end 14 integral therewith. The front side 18 is shown in FIG. 6 without the faceplate 20. As seen in FIG. 6A the first body portion 46 includes a rectangular recess disposed therein defined by a bottom wall 50, two side walls 52a, 52b, a front wall 54, and two angled walls 56a, 56b connecting the side walls 52a, 52b, respectively, with the front wall 54. Walls 58a-58c define a rectangular cut-out portion 59 disposed in the front wall 54. The second body portion 48, as shown in FIGS. 7 and 7A, forms the rear side 34 of the cylindrical body 10 and a further portion of the bottom end 14 integral therewith. FIG. 7A shows a front view of the second body portion 48 including a rectangular recess disposed therein defined by a bottom wall 60, two side walls 62a, 62b, an intermediate wall 64, and two angled walls 66a, 66b connecting the side walls 62a, 62b, respectively, with the intermediate, wall 64. The intermediate wall 64 further has a channel 68 disposed therein. The channel 68 is defined by a second pair of angled side walls 70a, 70b, a rear wall 72, a portion of the bottom wall 60 and an opposing top wall 74. The intermediate wall 64 and the second pair of angled walls 70 substantially define the first and second elongate portions 38, 40 as shown in the rear view of the second body portion 48 depicted in FIG. 7.

Referring to FIGS. 6A, 7A, and 9, the first arid second body portions 46, 48 include aligned bores 75 within which tapered pins 77 are press-fitted. In one arrangement, the tapered pins 77 are sized relative to the bores 75 and function alone to hold the first and second body portions 46, 48 together in a permanent fashion. Alternatively, the pins 77 and an adhesive or other securing means may be used to hold the body portions 46, 48 together in either a permanent or semi-permanent fashion. Specifically, FIGS. 6A and 7A depict bores 75a-75h and bores 75i-75p, respectively, which are aligned with one another after a chassis 76 is placed therebetween (FIGS. 8, 9, and 9A) during assembly of the body 10. Tapered pins 77 (only pins 77a-77d are shown in phantom in FIG. 9) are disposed within the holes 75a-75p to secure the first and second body portions 46, 48 together as noted above. As provided within the second chamber 106. The battery contact holders 108 are configured to press conductive leads. 110 against ends of standard AA batteries (not shown). The leads 110 extend from ends of the batteries to the circuit, thereby providing power to same.

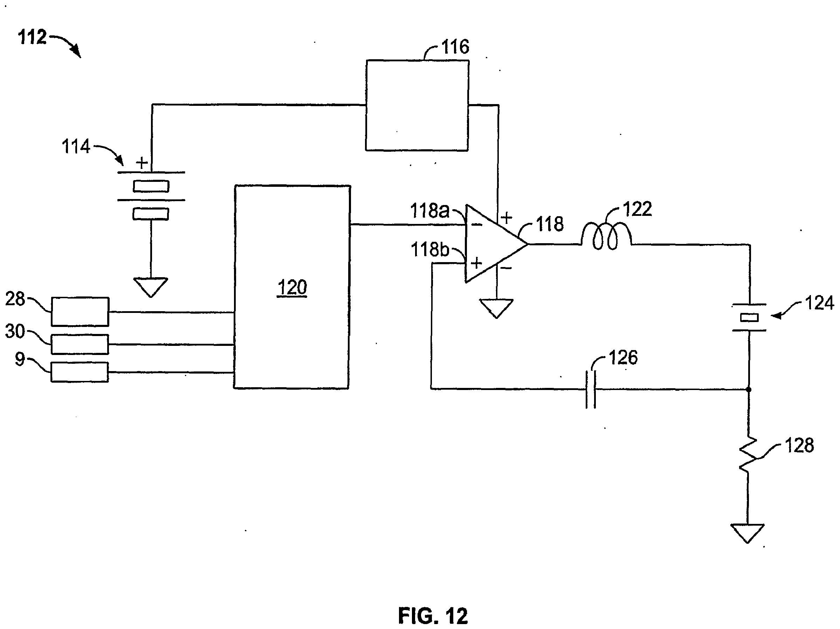

Referring next to FIG. 12, a control circuit 112 for driving the fluid emitting device 2 includes a d. c. power supply 114 that is electrically connected to a voltage regulator 116. The voltage regulator 116 supplies electrical power to an output driver 118. Output driver 118 may be an amplifier of any suitable type, the details of which are readily apparent to a person of ordinary skill in the art The control circuit 112 further includes a digital input and control block 120 that is connected to a first input 118a of the output driver 118 to regulate the operation of the fluid emitting device in accordance with the position of the dial 28 and/or the activation button 30 and or the motion sensor 9 as described in detail hereinafter. The output driver 118 controls the flow of electric current through an inductor 122 that is connected in series between the output driver 118 and a piezoelectric element 124. A feedback capacitor 126 is connected between the piezoelectric element 124 and a second input 118b of the output driver 118. In addition a resistor 128 is connected between control circuit 112 and ground at a junction between the piezoelectric element 124 and the feedback capacitor 126. The control circuit 112 causes current to flow through the piezoelectric element 124 at a resonant frequency determined by, among other things, the characteristic impedance of the element 124, the impedance of the inductor 122, and the temperature of the element 124. The feedback capacitor 126 advances the phase of the feedback signal by 90 degrees as required for proper operation.

If desired, one could use the circuit shown in Nakane et al. U. S. Patent No. 4,632,311 to drive the piezoelectric element 124 at the resonant frequency.

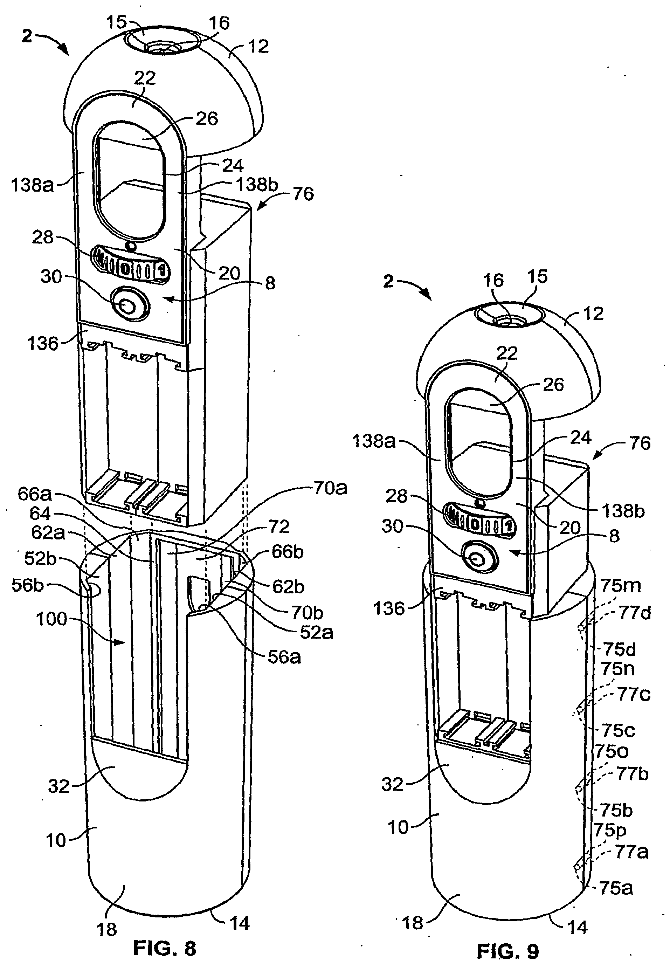

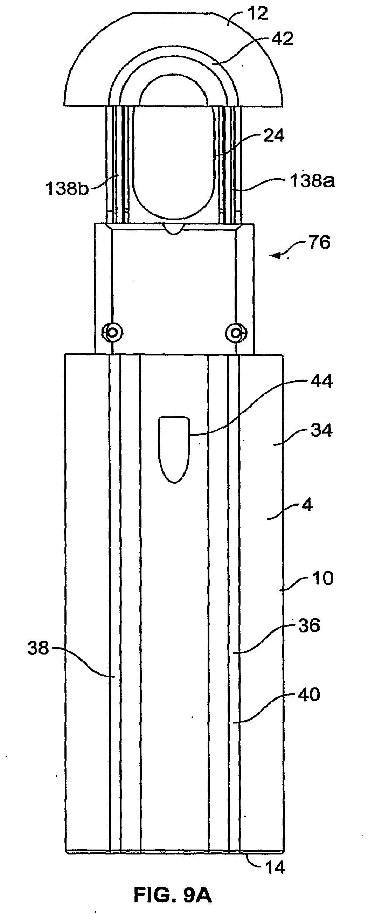

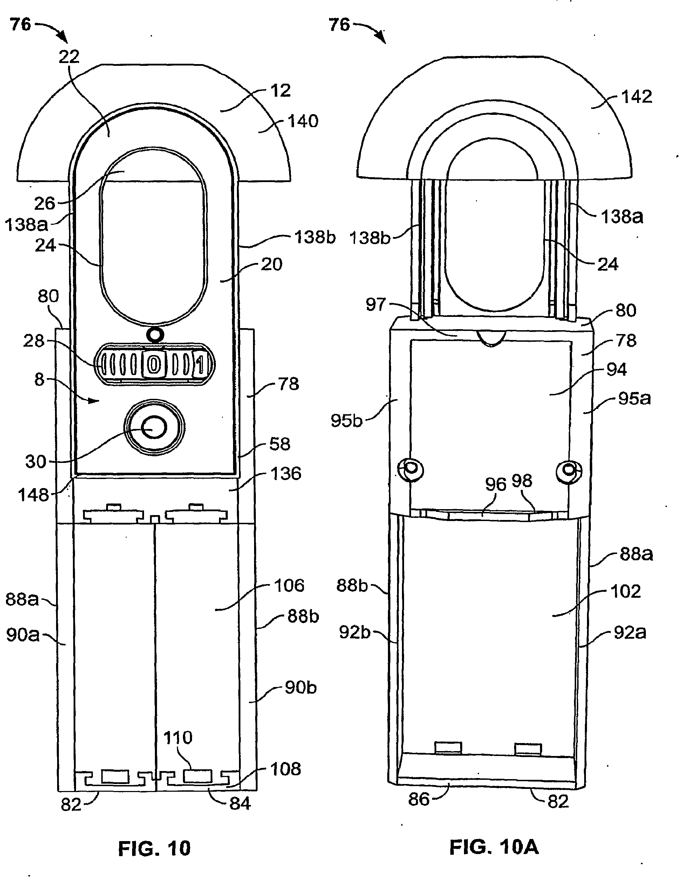

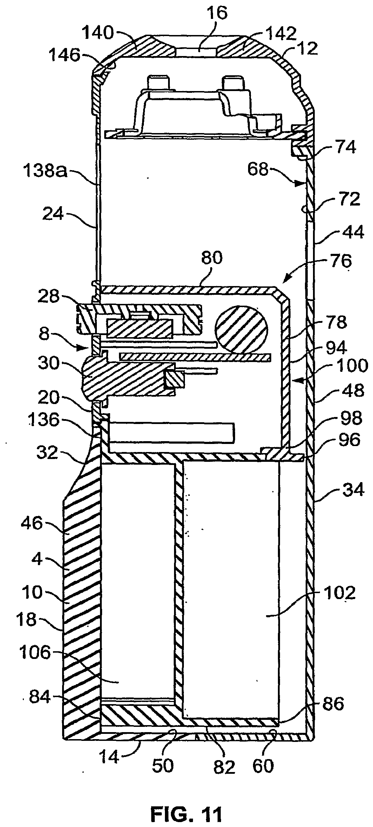

The chassis 76 further includes a rectangular portion 136 (FIGS. 8-10) that extends outwardly from the front wall 84 of the block 78 above the second chamber 106. Two aims 138a, 138b extend between the top wall 80 and the hemispherical top portion 12 of the housing 4. The top portion 12 comprises a first top portion 140 and a second top portion 142 (FIGS. 10 and 11). The first top portion 140 includes bores 144a, 144b that are aligned with bores 144c, 144d, respectively, of the second top portion 142. Tapered pins (not shown) similar to those discussed above are disposed in the aligned bores 144a-d in the portions 140, 142 and similarly secure the first and second top portions 140, 142 together. The first top portion 140 is integrally attached to an inner side 146 of the arms 138a, 138b in spaced relation from the top wall 80 of the block 78. Further, the top portion 12 is centered above the top wall 80 of the block 78. A recess within the rectangular portion 136, the arms 138a, 138b, and the first top portion 140 is sized to allow the faceplate 20 to be secured therein. The faceplate 20 is recessed an appropriate distance so as to be flush with the rectangular cut-out portion 58 of the body 10. When the chassis 76 is moved from the open to the closed position, a lower end 148 of the faceplate contacts the body 10, thereby preventing further movement of the chassis 76.

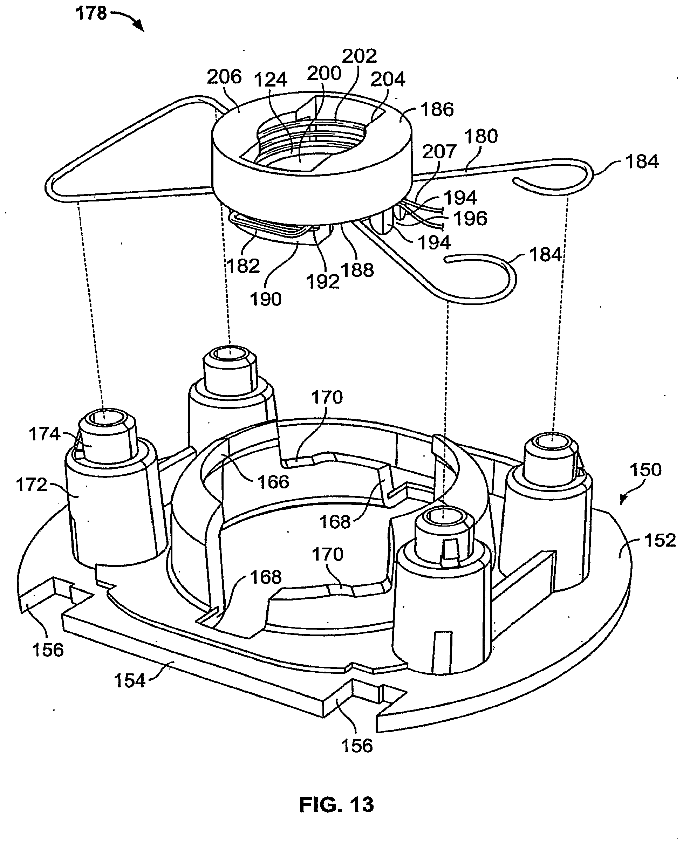

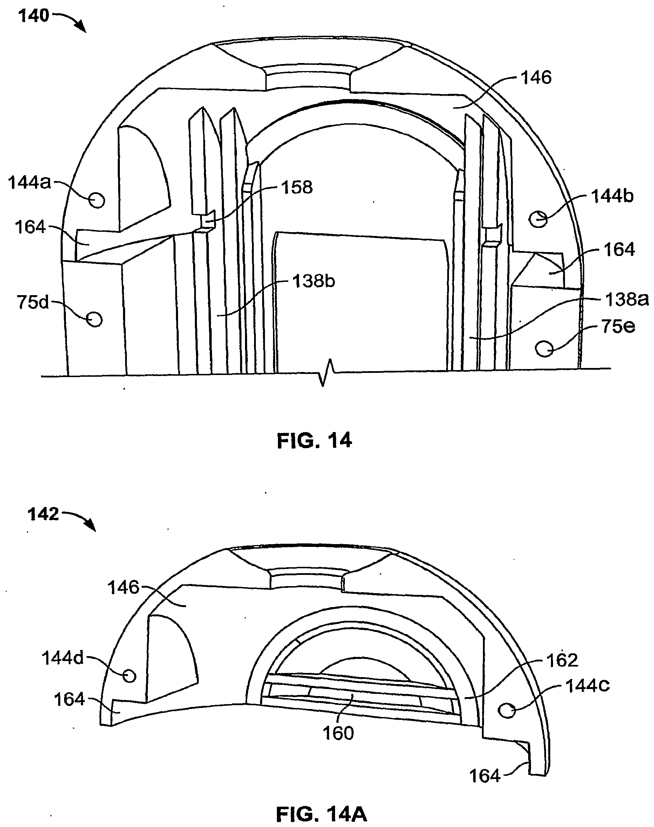

A support chassis 150 useful in the present arrangement includes those described in, e.g., U.S. Patent No. 6,896,193. In an arrangement depicted in FIG. 13 the support chassis 150 is provided within the top portion 12, and is shown in further detail in FIGS. 14 and 14A. The support chassis 150 comprises an oval base plate 152 that is truncated on one side to form a flat end 154. The flat end 154 includes two slots 156 that engage with corresponding slots 158 within the arms 138 of the chassis 76 as shown in FIG. 14. The portions of the arms 138 having the slots 158 are disposed on the inner side 146 of the first top portion 140. A side of the base plate 152 opposite the flat end 154 is recessed within a groove 160 formed into a protrusion 162. The protrusion 162 extends from the inner side 146 of the second top portion 142 as shown in FIG. 14A. An outer periphery of the base plate 152 between the flat end 154 and the opposite side is in contact with two opposing walls 164 that depend from the inner side 146 of the top portion 12. The support chassis 150 further comprises an upwardly extending cylindrically shaped reservoir mounting wall 166. Two opposing bayonet slots 168 are formed into the reservoir mounting wall 166 and the base plate 152. Each bayonet slot 168 includes a circumferentially extending detent 170. Four cylindrical projections 172 extend upwardly from the base plate 152, wherein each projection 172 includes a smaller second projection 174 extending from a top end thereof.

FIG. 13 also shows a piezoelectric actuator and orifice plate assembly 178 similar to those described in U.S. Patent No. 6,896,193. The assembly 178 includes a metal wire frame 180. The metal wire frame 180 has a flat end with opposing sides of the frame 180 extending inwardly in an inverted V-shaped manner toward a central portion 182. The central portion 182 is defined by two opposing U-shaped sections. The two sides thereafter flare outwardly in a V-shaped manner with loops 184 at ends thereof. The central portion 182 of the metal frame 180 is attached to a hollow cylindrical assembly housing 186. A bottom end 188 of the assembly housing 186 includes two opposing inwardly stepped members 190 depending from the bottom end 188 thereof. Each stepped member 190 has a slot 192 disposed therein for the U-shaped sections of the metal frame 180 to extend through. Two protrusions 194 also depend from the bottom end 188 of the assembly housing 186 on one of the sides between the two opposing stepped members 190. A groove 196 is formed between the two protrusions 194. The frame 180 is disposed on the support chassis 150 by pressing the inverted V-shaped portion of the frame 180 adjacent the flat end around two of the projections 174 and the remaining two projections 174 into the loops 184.

The assembly 178 further includes the piezoelectric element 124 having an orifice plate 200 extending thereacross. The present piezoelectric element 124 is annular shaped and is disposed within the assembly housing 186 so that it rests upon the frame 180 adjacent the bottom end 188 of the assembly housing 186. The piezoelectric element 124 is held against the frame 180 by a spring 202 that is fitted into the assembly housing 186 between the piezoelectric element 124 and a truncated annular portion 204 that extends inwardly from a top end 206 of the assembly housing 186. Two wires 207 extend from the piezoelectric element 124 through the groove 196 and to the circuit. The wires 207 are provided to supply alternating electrical fields or voltages produced by the circuit to opposite sides of the piezoelectric element 124. When high frequency alternating electric fields are applied to the piezoelectric element 124 same undergoes changes to some of its physical dimensions. In
this arrangement, supplying alternating electric fields to the piezoelectric element 124 causes the diameter of the element 124 to alternatively decrease and increase, thereby causing the orifice plate 200 to vibrate up and down, respectively. Various piezoelectric mechanisms known to those skilled in the art may be utilized to produce a similar effect.

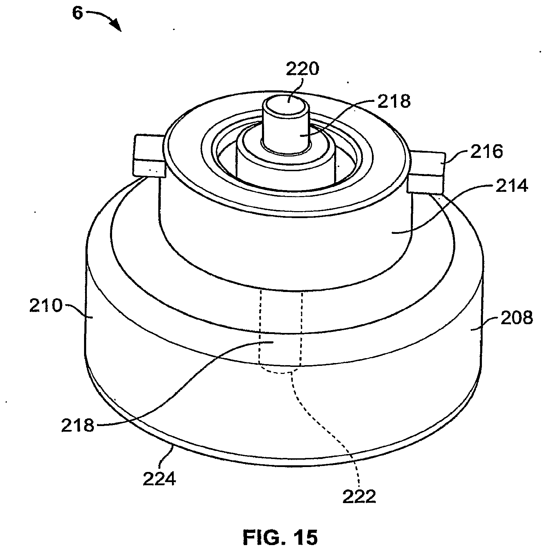

As noted above, the fluid reservoir 6 is removably inserted into the device 2 and may be fashioned in any manner known to those skilled in the art The present arrangement, as depicted in FIG. 15, utilizes a liquid reservoir 208. Other liquid reservoirs useful in the present embodiment include those disclosed in, e.g., U.S. Patent No. 6,293,474. The liquid reservoir 208 comprises a transparent cylindrical container 210 with a neck (not shown). A combination plug and wick holder 214 is affixed to the neck. The plug and wick holder 214 includes a pair of laterally extending mounting lugs 216. The liquid reservoir 208 is inserted into the support chassis 150 by aligning the lugs 216 with the bayonet slots 168 of the support chassis 150 and pressing the reservoir 6 upwardly, thereby inserting the lugs 216 into their respective bayonet slot 168. The liquid reservoir 208 is thereafter rotated counter-clockwise to force the lugs 216 to engage with the detents 170 of the respective bayonet slot 168 to secure the reservoir 208 within the device 2. The liquid reservoir 208 may be removed from the support chassis 150 by rotating the reservoir 208 clockwise to disengage the lugs 216 from their respective detents 170 and pulling the reservoir 208 downwardly so that the lugs 216 may pass through the bayonet slots 168. In this manner, the liquid reservoir 208 may be easily inserted into or removed from the device 2 when the device 2 is in the open position. The level of liquid within the liquid reservoir 208 may be monitored through the race-track shaped opening 24 in the faceplate 20 to provide a user an indication of whether the liquid reservoir 208 is in a first condition or in a second condition. For example, the first condition may indicate that the liquid reservoir 208 is full and the second condition may indicate that the liquid reservoir 208 is empty or nearly empty. Therefore, the opening 24 in the faceplate 20 and the liquid reservoir 208 act as an indicating system by informing a user whether the liquid reservoir 208 should be replaced or refilled.

A wick 218 is held within the combination plug and wick holder 214. An upper end 220 of the wick 218 extends beyond the neck and a lower end 222 of the wick 218 depends into the container 210 toward a bottom surface 224 thereof. The wick 218 transfers liquid by capillary action from within the reservoir 208 to the upper end 220 of the wick 218. The upper end 220 of the wick 218 is disposed adjacent a bottom of the orifice plate 200. During operation of the device 2 the orifice plate 200 vibrates up and down adjacent the upper end 220 of the wick 218. The up and down vibrations of the orifice plate 200 cause the liquid to be pumped through minute orifices in the orifice plate 200. Each orifice has a diameter within a range of about four microns to about ten microns. Alternatively, a discharge plate may be provided with a varying number of orifices and/or orifices having a different diameter. The support chassis 150 and the liquid reservoir 208 are adequately configured to ensure that the upper end 220 of the wick 218 does not apply an appreciable force to the orifice plate 200, thereby allowing liquid to be supplied to the orifice plate 200 without damping the vibrations of the plate 200 and reducing the effectiveness in atomizing the liquid. The pumping of the fluid through the orifice plate 200 causes the fluid to be ejected from a top of the orifice plate 200 in the form of aerosolized or atomized liquid particles. The atomized liquid particles thereafter traverse an unobstructed interior of the assembly housing 186 and pass through the liquid outlet 16 in the top end 16 of the housing 4. Thus, the liquid from the liquid reservoir 208 is discharged upwardly through the liquid outlet 16 and into the atmosphere. If desired, the liquid outlet 16 may instead be L-shaped or have any other nonlinear shape to direct the contents of the liquid reservoir 208 in a direction other than upwards. Still further, the cross-sectional shape and/or diameter of the liquid outlet 16 may be modified to obtain any desired spray pattern, or to alter the swirling and/or mechanical breakup of the discharged liquid, as should be evident to one of ordinary skill in the art.

The circuitry of the device 2 is activated by manipulation of the dial 28 and activation button 30 on the control panel 8. When the dial 28 is fully rotated to the left (clockwise) the device 2 is in an off state. Rotating the dial 28 to the right (counter-clockwise) away from the off position to an active position causes the device 2 to be in an activated state. When the dial 28 is in an active position, the device 2 operates in an automatic timed mode of operation as noted in greater detail below. Depression of the activation button 30 causes a manual spraying operation to be undertaken. The manual spraying option allows the user to override and/or supplement the automatic operation of the device 2 when so desired. Numerous other interfaces that have similar functional characteristics as described above may be provided on the control panel 8 or elsewhere on the device 2 and are intended to be within the scope of the present disclosure.

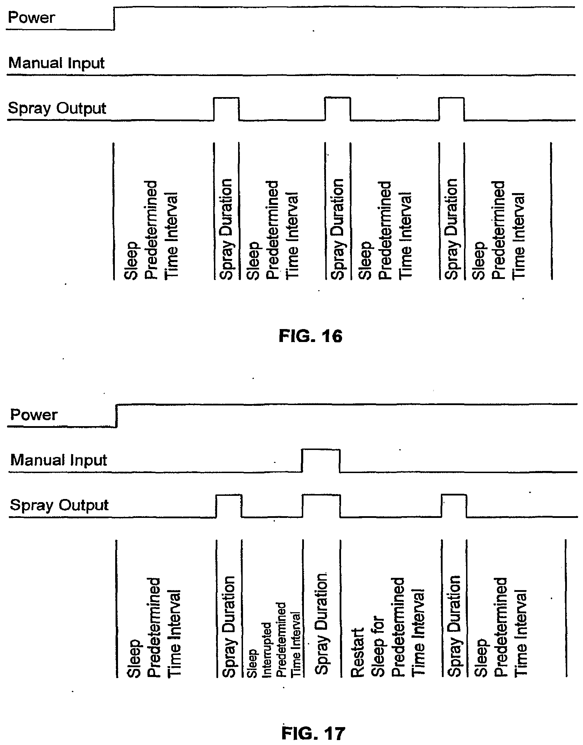

FIG. 16 depicts a timing diagram that illustrates the operation of the device 2. Initially, the device 2 is energized by moving the dial 28 from the off position to an active position by rotating the dial 28 to the right. The device 2 thereafter enters a first sleep period that lasts a predetermined time interval, such as about 1 hour. Upon expiration of the first sleep period the piezoelectric element 124 is activated to dispense fluid from the device 2 during a first spraying period. The first spraying period may last anywhere from a fraction of a second to a couple of seconds or longer. Automatic operation thereafter continues with alternating sleep and spraying periods.

The dial 28 provides for an infinite number of duty cycles. Rotating the dial 28 slightly to the right from the off position causes the device to have a sleep period of several hours. Rotating the dial 28 farther to the right reduces the sleep period, such as to an hour or a half hour. Additional rotation to the right further reduces the sleep periods to a couple of minutes or even a couple of seconds or less. If the dial 28 is completely rotated to the right, the sleep period is reduced to zero and the device 2 continuously sprays. The user may adjust the dial 28 to change future or current sleep periods at any time. The device 2 therefore allows for the piezoelectric element 124 to atomize the fluid during spray periods separated in time by sleep periods of adjustable durations.

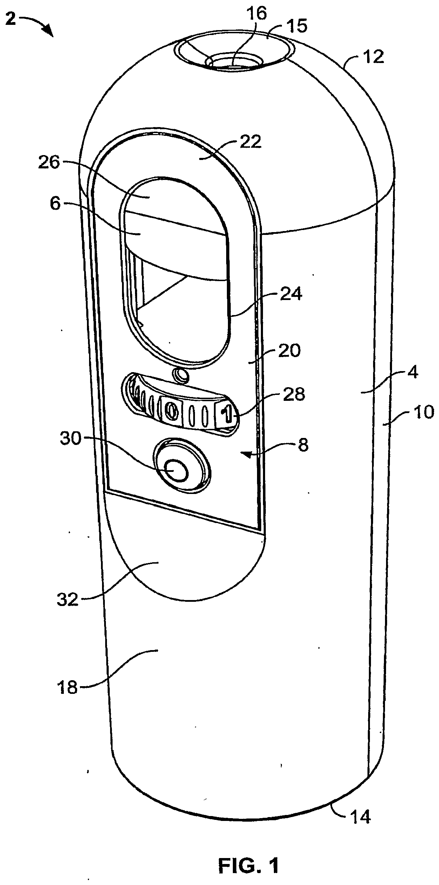

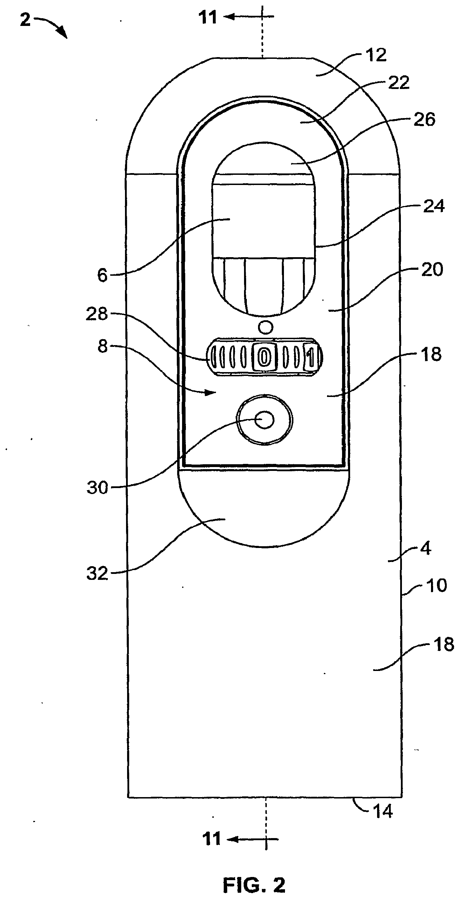

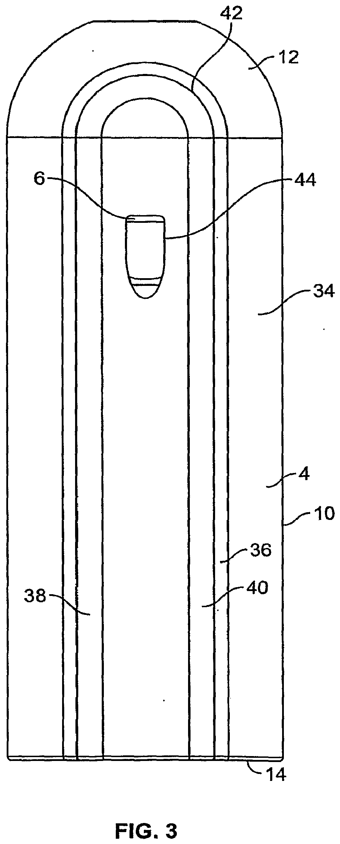

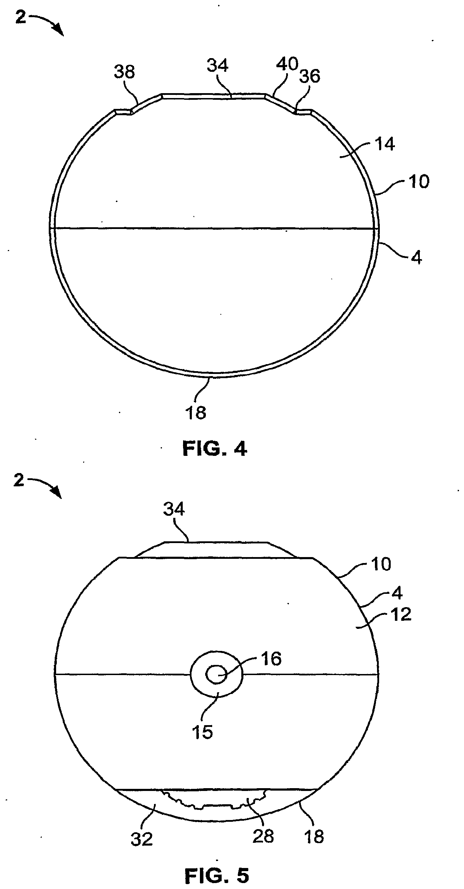

In one arrangement, the dial 28 is provided with visible numeric indicators associated with a zero position, a first position, a second position, a third position, a fourth position, and a fifth position. When the dial 28 is in the zero position the device 2 is in an off state. Rotating the dial 28 to the left (as seen in FIG. 1) to the first position causes the device 2 to alternate between a 22 second sleep period (or dwell time interval) and a 12ms puff of the fluid. The second, third, fourth, and fifth positions similarly result in emission of fluid for 12 ms following sleep periods (i.e., dwell time intervals) of 18 seconds, 13 seconds, 9 seconds, and 5 seconds, respectively. In a different arrangement, the dial 28 is provided with non-numeric indicators, such as lines, letters, icons, or the like. Further, any of the embodiments disclosed herein may have the ability to provide an infinite number of continuously variable spray duty cycles dependent upon the position of the dial 28.

FIG. 17 is similar to FIG. 16 except that the activation button 30 is depressed and released during the second sleep period. Momentarily depressing and releasing the activation button 30 causes the piezoelectric element 124 to dispense atomized fluid from the device 2 for either a fixed spray period, such as about one to seven seconds, or for a period of time dependent upon the length of time the button 30 is depressed. Upon completion of the spray period a third sleep period is entered into that lasts for the predetermined time interval. Automatic operation thereafter continues with alternating sleep and spraying periods. At any time during a sleep period, the user can manually activate the device 2 by depressing the activation button 30 as noted above. If the activation button 30 is depressed and held in a depressed state by the user for longer than a moment, the piezoelectric element 109 dispenses atomized fluid continuously until the activation button is released. Upon release of the button 30 a new sleep period is initiated that lasts for the predetermined time interval.

In any of the arrangements disclosed herein, the sleep periods may all be of the same duration, whether the spray operation is initiated manually or automatically. Also, in other arrangements the lengths of the automatic spray periods are all equal. If desired, one or more of the sleep periods may be longer or shorter than other sleep periods and/or one or more of the automatic spray periods may be longer or shorter than other spray periods. The lengths of the automatic spray periods may last anywhere from a fraction of a second to a couple of seconds or longer. The automatic spray periods may be modified to last even longer, such as until the complete exhaustion of the fluid in the device 2, or to comprise several sequenced discharges of the fluid. Still further, the control methodology can be modified to cause spraying operations to be periodically undertaken at equal or unequal intervals without regard to whether a manual spraying operation has been undertaken.

The present device 2 may be combined with the sensor 9 for activating the piezoelectric element 124. The device 2 may operate in a sensor mode and only activate the piezoelectric element 124 in response to output from the sensor 9. The device 2 could also operate in a combined timed and sensing mode of operation, wherein the piezoelectric element 124 is activated after completion of a sleep period or in response to output from the sensor 9. Following actuation of the piezoelectric element 124 by an output signal developed by the sensor 9, a new sleep period lasting the predetermined time interval may be initiated. In any of these embodiments the activation button 30 may be used to interrupt a sleep period with a manually activated spray period. The sensor 9 may be a motion sensor, a sound activated sensor, a light sensor, a temperature sensor, a vibration sensor, a malodorous compound detecting sensor, etc. In a particular arrangement, the sensor 9 comprises a photocell motion sensor that collects ambient light and allows a controller to detect any changes in the intensity thereof. It should be noted that numerous other motion sensors such as passive infrared or pyroelectric motion sensors, infrared reflective motion sensors, ultrasonic motion sensors, or radar or microwave radio motion sensors may be used with the present embodiment. In one arrangement only a single one of these sensors is utilized, while in other arrangements a combination of sensors is used. Further, the present listing of potential sensors is not exhaustive but is merely illustrative of the different types of sensors that can be used with the device 2 described herein. Still further, the placement of the device 2 is not confined to any of the specific examples described above. It is intended that the device 2 be placed in any area where the dispensing of an atomized fluid is required or desired and/or where the sensor 9 is effective.

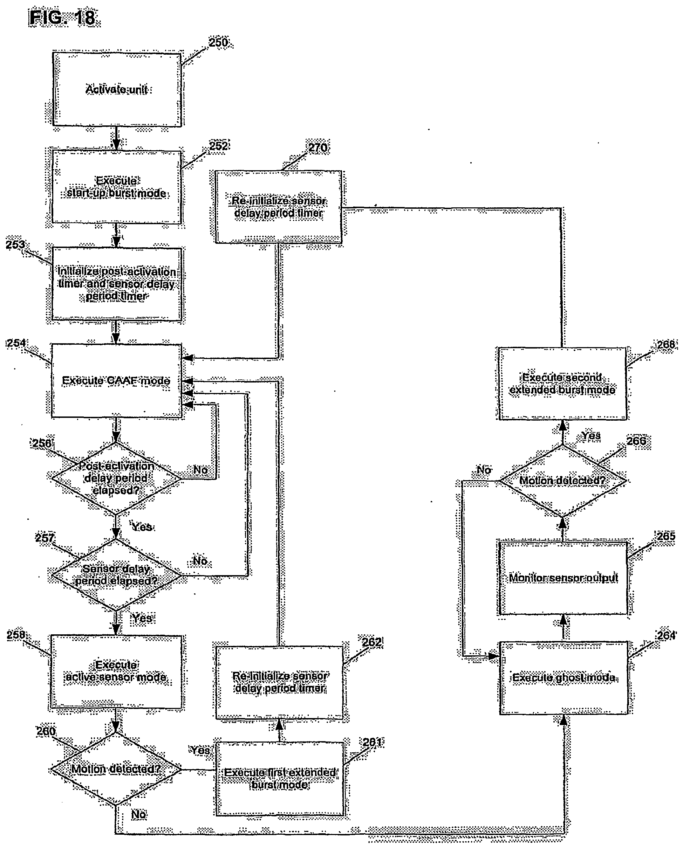

FIG. 18 depicts a block diagram illustrating the functioning of one arrangement of the device 2. For purposes of the present example, it is assumed that the inactive device 2 is placed in a room such as a household bathroom. A block 250 activates the device 2 when the dial 28 is rotated to the left as seen in FIG. 1 from the zero position to one of the first to fifth active positions. It will further be assumed for purposes of the present example that the dial 28 has been rotated to the first position to initiate an automatic spray time interval of about 22 seconds.

A block 252 then initiates a start-up burst mode upon activation of the device 2. The start-up burst mode provides an initial burst of fluid from the device 2 upon expiration of a dwell period or interval following energization of the device 2. The start-up burst mode provides for a dwell interval of about 1 second to about 2 minutes following movement of the dial 28 from the zero position to any of the remaining positions. In a preferred arrangement the dwell interval elapses after about a minute. After the dwell interval has elapsed, fluid is emitted during a start-up spray period of about one second to about ten seconds, and more preferably for about three seconds. The piezoelectric element 124 is operated during this start up spray period by alternatively energizing and deenergizing the element 124 in 12 ms 50% duty cycles.

A block 253 initializes and starts a post-activation timer and a sensor delay period timer. The post-activation timer starts running upon activation of the device 2 and is activated only once immediately following each energization of the device 2. The post-activation timer indicates when a post-activation delay period has elapsed. The post-activation delay period lasts for a specified interval, such as 15 minutes. The sensor delay period timer is utilized to determine when a sensor delay period has elapsed.

Thereafter, a block 254 executes a continuous action air freshener (hereinafter "CAAF") mode of operation. The CAAF mode provides for timed bursts of fluid dependent on the position of the dial 28. In the present example, a 12 ms burst of fluid is dispensed every 22 seconds. The timing of the CAAF mode may be modified by adjusting the dial 28 to increase or decrease the time between bursts of the fluid.

A query is undertaken during the CAAF mode at a block 256, which determines whether the post activation delay period has elapsed. If the 15 minute post activation delay period has not elapsed the CAAF mode continues uninterrupted. Once the 15 minute post activation period has elapsed a second query is undertaken by a block 257 that determines whether the sensor delay period has elapsed. In the preferred arrangement, the sensor delay period timer senses a 15 minute period of time during which the signal developed by the sensor 9 is ignored. If the 15 minute sensor delay period has not elapsed the CAAF mode continues uninterrupted; however, if the sensor delay period has elapsed the device 2 enters an active sensor mode implemented by a block 258.

As illustrated, the sensor 9 comprises a photocell motion sensor that collects ambient light and allows a controller to detect any changes in the intensity thereof. The active sensor mode causes the device 2 to register the signal developed by the sensor 9. A block 260 undertakes a query to determine whether the registered signal indicates that motion has been detected by the sensor 9. If motion is detected by the sensor 9 a block 261 executes a first extended burst mode. The first extended burst mode causes the device 2 to emit an extended burst of fluid for a specified duration independent of the dial 28. In the present example, the first extended burst of fluid lasts for a period of three seconds and is emitted by alternatively energizing and deenergizing the element 124 in 12 ms 50% duty cycles. Thereafter, a block 262 re-initializes and restarts the sensor delay period timer and the CAAF mode is resumed. If motion is not detected by the sensor 9 a block 264 executes a ghost mode of operation. The ghost mode conserves fluid by providing timed bursts of fluid independent of the position of the dial 28. In one arrangement, the ghost mode emits timed bursts of fluid separated by a dwell period or interval greater than the user-selectable dwell periods or intervals of the CAAF mode. Alternatively or in addition, the device 2 may emit shorter bursts of fluid during operation in the ghost mode than are emitted during operation of the CAAF mode. In the present example, a 12 ms burst of fluid is dispensed every three minutes regardless of the position of the dial 28 during operation in the ghost mode. The active life span of the fluid in the reservoir 6 is therefore extended by decreasing the frequency and/or duration of bursts of fluid during periods of little or no activity.

During the ghost mode of operation a block 265 continuously registers the signal developed by the sensor 9 and a block 266 continuously queries whether the signal developed by the sensor 9 indicates that motion has occurred. If the block 266 determines that no motion has occurred the ghost mode continues uninterrupted. However, if the block 266 determines that motion has occurred a block 268 executes a second extended burst mode.

The second extended burst mode causes the device 2 to emit an extended burst of fluid for a specified duration independent of the dial 28. In the present example, the extended burst of fluid lasts for a period of three seconds and is emitted by alternatively energizing and deenergizing the element 124 in 12 ms 50% duty cycles. Thereafter, a block 270 re-initializes and restarts the sensor delay period timer and the block 254 thereafter resumes the CAAF mode of operation.

At any time during the active state of the device 2 the activation button 30 may be depressed to initiate a manual burst mode. The manual burst mode allows the user to emit fluid from the device 2 regardless of the position of the dial 28 or what mode the device 2 is currently in. In the present example, when the activation button 30 is depressed for less than a second the fluid is emitted for a pre-set period of time of about three seconds. Alternatively, if the activation button 30 is depressed for more than a second the fluid is emitted continuously for a user determined period time that ceases when the user releases the activation button 30. The piezoelectric element 124 is operated during the manual burst mode by alternatively energizing and deenergizing the element 124 in 12 ms 50% duty cycles for the pre-set period of time or the user determined period. The duration that the activation button 30 must be pressed to activate the user determined period may be modified. Similarly, the duration that the pre-set period lasts or the timing interval used to alternatively energize and deenergize the element 124 may be modified as well. Further, it is anticipated that any of the spray periods or delay periods discussed in connection with the present arrangement may be likewise modified.

The present device 2 may also be used in a manner consistent with the use of commercially marketed hand-held aerosol containers. Rather than activating the device 2 by way of a timer or sensor, the device 2 may be kept in an inactive state until a user requires the fluid to be dispensed. The user picks up the device 2 by gripping the container body 10 with his or her hand. The device 2 is activated by the user depressing the activation button 30 with a thumb or finger. A single depression and release of the activation button 30 causes the piezoelectric element 124 to dispense atomized fluid from the device 2 for a limited spray period. In some arrangements the spray period will last for the same duration the button 30 is held down while in other embodiments the spray period may last for a fixed spray interval, such as several seconds.

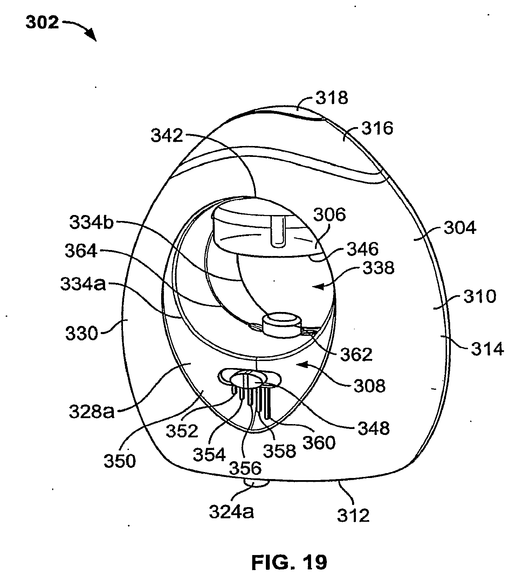

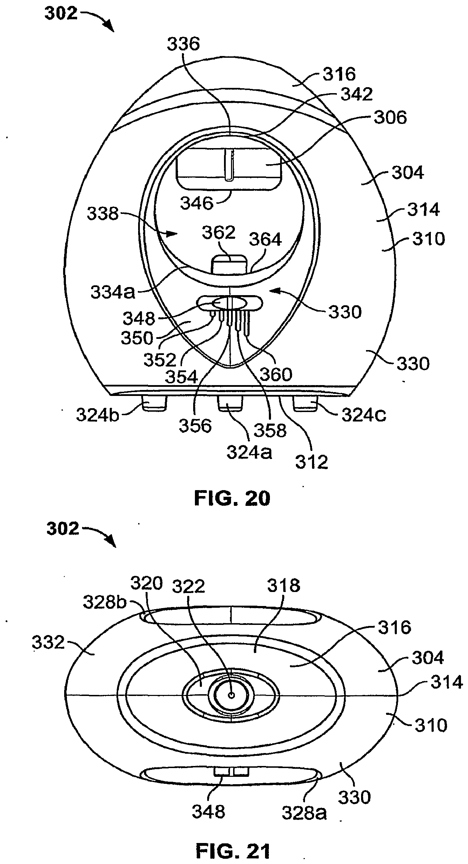

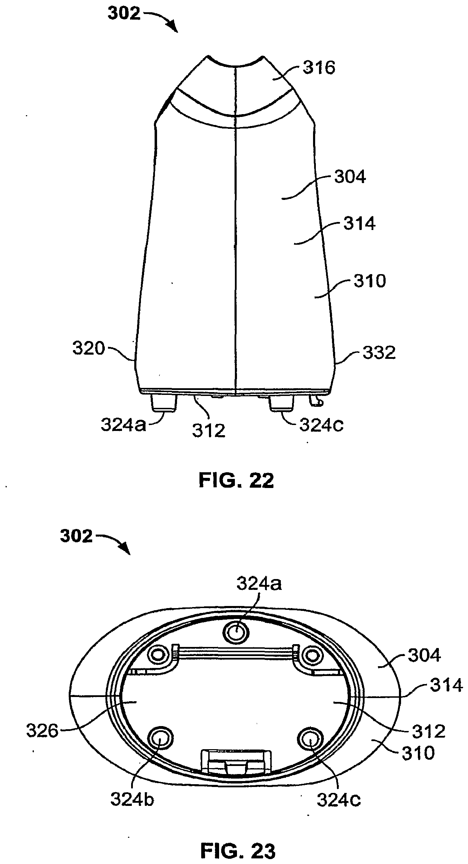

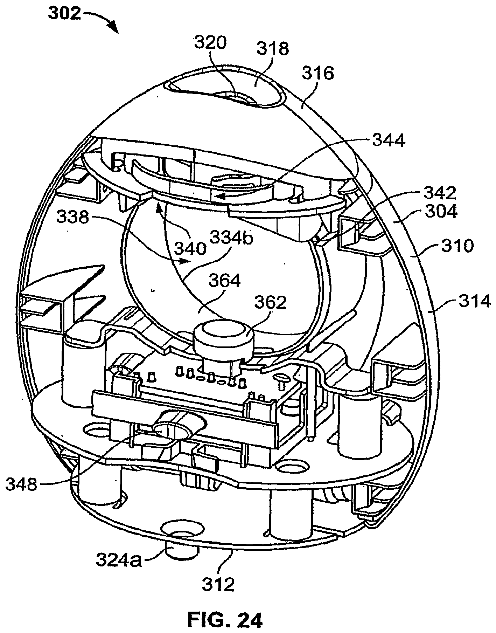

FIGS. 19-24 depict a arrangement of an atomizing device 302 similar in functionality and structure to the atomizing device 2 described herein. The device 302 generally comprises a housing 304, a fluid reservoir 306, and a control panel 308. The device 302 is operationally disposed on a support surface (not shown) and emits fluid upon receipt of an activation signal from an internal timer and/or sensor 309. The device 302 may also be manually activated via the control panel 308 to provide a burst of fluid. Similar to the device 2 described herein, the emitted fluid may be a fragrance, sanitizing agent, household cleaner, insecticide, or insect repellant disposed within a carrier liquid, a deodorizing liquid, or the like. The fluid alternatively comprises any fluid known to those skilled in the art that can be dispensed from a reservoir.

The housing 304 is typically made from a molded plastic, such as polypropylene. The housing 304 comprises an elliptical body 310 with a bottom surface 312, a central portion 314, and a top portion 316. In one particular arrangement, the body 310 has an overall height of about 109 mm (4.29 in.), an overall width of about 91 mm (3.58 in.), and an overall depth of about 54 mm (2.13 in.). The top portion 316 of the body 310 includes first and second elliptical recesses 318, 320, respectively, that taper inwardly toward a central circular outlet opening 322. The outlet opening 322 in one arrangement has a diameter of at least about 8 mm (0.32 in.). The bottom surface 312 of the body 310 is planar and includes three equidistantly placed cylindrical feet 324a, 324b, 324c disposed thereon. A door 326 is hingedly secured to the bottom surface 312 for movement between open and closed positions. When the door 326 is in an open position (not shown) battery terminals similar to those on the position of the switch 348. In one arrangement, manipulation of the switch 348 to the first position 352 causes a sleep period of 22.54 seconds, i.e., the device 302 has a dwell or sleep period of 22.54 seconds between activations of the piezoelectric element to dispense the fluid. A second position 354 of the switch 348 corresponds to a sleep period of 12.81 seconds, a third position 356 corresponds to a sleep period of 9.23 seconds, a fourth position 358 corresponds to a sleep period of 7.18 seconds, and a fifth position 360 corresponds to a sleep period of 5.65 seconds. The first through fifth positions 352-360 are indicated by markings beneath the switch 348. The user may adjust the switch 348 to change future or current sleep periods at any time. The device 302 therefore allows for the piezoelectric element within the assembly 344 to atomize the fluid during spray periods separated in time by sleep periods of adjustable amounts.

The device 302 also includes an instant action button 362 disposed on a lower wall 364 defining the cylindrical hollow portion 338. The button 362 is centered beneath the bottom surface 346 of the fluid reservoir 306. Depression of the button 362 causes.a manual spraying operation to be undertaken. The manual spraying option allows the user to override and/or supplement the automatic operation of the device 302 when so desired. The present button 362 acts in a similar manner as the activation button 30 described in connection with the device 2 and depicted in FIG. 17. Depressing and releasing the activation button 362 during a sleep period or automatic spray period causes the assembly 344 to dispense atomized fluid from the device 302 for a manual spray period, such as about one to five seconds. Upon completion of the manual spray period a sleep period is entered into that lasts for a predetermined time interval dependent on the position of the slide switch 348. Automatic operation thereafter continues with alternating sleep and spraying periods. At any time during a sleep period, the user can manually activate the device 302 by depressing the button 362 as noted above. In a further embodiment, if the button 362 is depressed and held in a depressed state by the user the piezoelectric element dispenses the atomized fluid continuously until the button 362 is released. Upon release of the button 362 a new sleep period will be initiated that will last for the predetermined time interval.

The structure and functionality described in connection with the device 302 is also intended to be used in connection with the device 2 in alternative arrangements thereof. Similarly, the arrangements described in connection with the device 2 may be alternatively used or modified with respect to the device 302. For example, the timing and duration of automatic or manual sleep and spray periods for the devices 2 and 302 may be utilized or adjusted in any manner described herein for either device. Other modifications contemplated with the present arrangements include supplying the device 302 with a sensor described in connection with the device 2 or providing the device 2 with a slide switch as opposed to the dial 28. Further, numerous other interfaces that have similar functional characteristics as described above may be provided on either of the devices 2 and 302 and are intended to be within the scope of the present disclosure. Still further, the present application contemplates variations to the structure of either of the devices 2, 302. For instance, the aerosolized liquid outlet 16 of the device 2 may be modified to direct or break up fluid passing therethrough in a desired manner known by those skilled in the art or the body 10 may be fashioned to comprise a different shape such as a rectangle, triangle, or oval. Those skilled in the art will realize the numerous manners in which the present disclosure may be modified to provide similar functionality to that already disclosed herein.

### INDUSTRIAL APPLICABILITY

One advantage of the present invention is the ability to remove the user's hand from an area adjacent the fluid being pumped from the device, thereby preventing residual fluid from settling onto the user's hand. This advantage is possible because the control panel that includes the activation button is disposed beneath the support chassis and the reservoir. Further, a user can readily determine the current quantity of the fluid in the reservoir without having to disassemble components. The device can be held in any orientation during spraying, and the sizes of the aerosolized droplets are significantly smaller than droplets emitted from conventional aerosol containers. This latter feature promotes dispersal of emitted fluid and minimizes undesirable fallout.

## Claims

1. A device for dispensing a fluid, comprising:
a housing (4) having an internal power source and a mounting assembly (150) adapted for receiving a replaceable fluid reservoir (6) that includes a capillary element (218) for movement of the fluid to a discharge end thereof;
a mechanism (124) within the housing (4) and energized by the internal power source for vibrating a perforated discharge plate (200) disposed adjacent the discharge end of the capillary element, the mechanism (124) providing sufficient vibratory movement in a dispensing state to pump the fluid from the discharge end through the discharge plate (200) and into the atmosphere; and
a control panel (8) carried by the housing (4) and disposed beneath the mounting assembly (150), wherein the control panel (8) provides an interface for a user to select at least one of a timed mode of operation, an automatic mode of operation dependent upon a sensor output developed by a sensor, and a manual mode of operation, and
wherein the mounting assembly (150) is further adapted to receive the replaceable fluid reservoir (6) in a manner that allows same to be visually inspected during an in-use condition;
wherein the interface comprises a manual activation button (30) for activating the mechanism, and
**characterized in that** said device is designed to cause upon depression of the activation button the mechanism (124) to dispense atomized fluid from the device during a manual spraying period of between about 1 second to about 7 seconds, and continuous depression of the manual activation button (30) for a specified duration causes the mechanism to dispense atomized fluid continuously until the manual activation button (30) is released.

2. The device of claim 1, wherein the replaceable fluid reservoir (6) is received within the mounting assembly (150).

3. The device of claim 2, wherein the replaceable fluid reservoir (6) provides an indication to the user on whether the fluid reservoir (6) should be replaced.

4. The device of claim 1, wherein the interface is positioned to allow the user to select the at least one of the timed mode of operation, the automatic mode of operation dependent upon the sensor output, and the manual mode of operation in a manner that does not obstruct the fluid pumped during the dispensing state.

5. The device of claim 1, wherein the interface comprises a dial that is rotatable between an active position and a non-active position; wherein rotation of the dial to a particular position establishes a dwell time interval during which the mechanism remains inactive, and wherein activation of the mechanism occurs upon expiration of the dwell time interval.

6. The device of claim 5, wherein the dwell time interval is between about 0 seconds and a continuously inactive spraying state.

7. The device of claim 5, wherein the mechanism is actuable during the dwell time interval by an activation signal.

8. The device of claim 7, wherein the activation signal is developed on the sensor output or is developed by manual activation of a switch.

9. The device of claim 1, wherein the sensor comprises at least one of a motion sensor and a malodorous compound detecting sensor.

10. The device of claim 1, wherein activation of the mechanism causes the fluid to be emitted during a particular spraying period.

11. The device of claim 10, wherein the spraying period has a duration between about one second and about seven seconds.

12. The device of claim 1, wherein the mechanism (124) for vibrating a perforated discharge plate (200) is a piezoelectric element.

13. A method for dispensing a fluid from a dispenser according to claim 1, comprising:
providing a housing (4) having an internal power source, a mounting assembly (150) for receiving a replaceable fluid reservoir (6), a mechanism (124) for vibrating a perforated discharge plate (200), and a control panel (8) for activating the dispenser; wherein the mechanism can be activated by at least one of a timed mode of operation, an automatic mode of operation dependent upon an output of a sensor, and a manual mode of operation,
retaining the replaceable fluid reservoir (6) within the mounting assembly (150), the fluid reservoir (6) including a capillary element for movement of a fluid to a discharge end thereof;
providing a fluid for dispensing within the replaceable fluid reservoir (6);
orienting the replaceable fluid reservoir (6) within the mounting assembly (150) to allow a user to determine a level of the fluid within the fluid reservoir (6);
activating the mechanism wherein activation causes the perforated discharge plate (200) to vibrate adjacent the discharge end of the capillary element and pump the fluid from the discharge plate and into the atmosphere; and
energizing the mechanism by the internal power source;
the method further comprising the steps of pressing a button on the control panel (8) longer than a predetermined period, which causes the mechanism to dispense fluid continuously until the button is released.

14. The method of claim 13, further including the step of monitoring the level of the fluid within the replaceable fluid reservoir to determine whether the fluid reservoir is in a first full condition or in a second refill condition.

## Patentansprüche

1. Gerät zum Ausgeben eines Fluids, mit:
einem Gehäuse (4) mit einer internen Stromquelle und einer Halterung (150) zur Aufnahme eines auswechselbaren Fluid-Vorratsbehälters (6), der ein Kapillarelement (18) zur Zufuhr des Fluids zu einem Ausgabeende aufweist;
einer im Gehäuse (4) angeordneten Mechanik (124), die von der internen Stromquelle betreibbar ist, um eine am Ausgabeende des Kapillarelements angeordnete perforierte Ausgabeplatte (200) in Schwingungen zu versetzen, wobei die Mechanik (124) in einem Ausgabezustand eine Schwingungsbewegung erzeugt, die ausreicht, um das Fluid vom Ausgabeende weg durch die Ausgabeplatte (200) und in die Umluft zu pumpen; und
einem Bedienfeld (8), das vom Gehäuse (4) getragen und unter der Halterung (150) angeordnet ist, wobei das Bedienfeld (8) eine Schnittstelle bereitstellt, an der ein Benutzer mindestens einen zeitgesteuerten Betrieb oder einen selbsttätigen, von einem Ausgangssignal eines Sensors entwickelten abhängigen oder einen manuellen Betrieb auswählen kann; und
wobei die Halterung (150) weiterhin ausgeführt ist, das auswechselbare Fluidreservoir (6) so aufzunehmen, dass es im Gebrauch sichtprüfbar ist,
wobei die Schnittstelle zum Aktivieren der Mechanik einen Taster (30) zur Handbetätigung aufweist;
**dadurch gekennzeichnet, dass** das Gerät so konstruiert ist, dass beim Drücken des Aktivierungstasters die Mechanik (124) zerstäubtes Fluid für ein Hand-Sprühintervalls zwischen etwa einer und etwa 7 Sekunden Länge auszugeben, und dass beim stetigen Drücken des Hand-Aktivierungstasters (30) für eine bestimmte Dauer die Mechanik zerstäubtes Fluid stetig ausgibt, bis der Hand-Betätigungstaster (30) freigegeben wird.

2. Gerät nach Anspruch 1, dessen auswechselbarer Fluid-Vorratsbehälter (6) in der Halterung (150) aufgenommen wird.

3. Gerät nach Anspruch 2, dessen auswechselbarer Fluid-Vorratsbehälter (6) dem Benutzer eine Anzeige liefert, ob der Fluid-Vorratsbehälter (6) ausgewechselt werden sollte.

4. Gerät nach Anspruch 1, dessen Schnittstelle so angeordnet ist, dass der Benutzer den zeitgesteuerten, den vom Sensor-Ausgangssignal abhängigen selbsttätigen oder den Handbetrieb auf eine Weise auswählen kann, dass das im Ausgabezustand gepumpte Fluid nicht behindert wird.

5. Gerät nach Anspruch 1, dessen Schnittstelle eine Skala aufweist, die zwischen einer aktiven und einer inaktiven Stellung drehbar ist, wobei die Drehung der Skala in eine bestimme Lage ein Verweilintervall festlegt, während dessen die Mechanik inaktiv bleibt und mit dessen Ablauf die Mechanik aktiviert wird.

6. Gerät nach Anspruch 5, bei dem das Verweilintervalls zwischen etwa null Sekunden und einem ständig inaktiven Sprühzustand liegt.

7. Gerät nach Anspruch 5, bei dem die Mechanik während des Verweilintervalls durch ein Aktivierungssignal aktivierbar ist.

8. Gerät nach Anspruch 7, bei dem das Aktivierungssignal vom Sensor-Ausgangssignal oder von der Handbetätigung eines Schalters abgeleitet wird.

9. Gerät nach Anspruch 1, dessen Sensor mindestens ein Bewegungssensor oder ein Sensor ist, mit dem eine übelriechende Verbindung erfassbar ist.

10. Gerät nach Anspruch 1, bei dem durch Aktivieren der Mechanik das Fluid während eines bestimmten Sprühintervalls ausgegeben wird.

11. Gerät nach Anspruch 10, bei dem das Sprühintervall zwischen etwa einer Sekunde und etwa sieben Sekunden dauert.

12. Gerät nach Anspruch 1, bei dem die Mechanik (24) zum Schwingenlassen einer perforierten Ausgabeplatte (200) ein piezoelektrisches Element ist.

13. Verfahren zum Ausgeben eines Fluids aus einem Gerät nach Anspruch 1 mit folgenden Schritten:
Bereitstellen eines Gehäuses (4) mit einer internen Stromquelle, einer Halterung (150) zur Aufnahme eines auswechselbaren Fluid-Vorratsbehälters (6), einer Mechanik (124) zum Schwingenlassen einer perforierten Ausgabeplatte (200) und einem Bedienfeld (8) zum Aktivieren des Geräts, wobei die Mechanik zu mindestens einem zeitgesteuerten Betrieb oder einem selbsttätigen, von einem Ausgangssignal eines Sensors entwickelten Ausgangssignal abhängigen Betrieb oder zu einen manuellen Betrieb aktivierbar ist;
Einbringen des auswechselbaren Fluid-Vorratsbehälters (6) in die Halterung (150), wobei der Fluid-Vorratsbehälter (6) ein Kapillarelement zur Bewegung eines Fluids zu einem Ausgabeende desselben aufweist;
Bereitstellen eines Fluids in dem auswechselbaren Fluid-Vorratsbehälter (6) zwecks Ausgabe;
Ausrichten des auswechselbaren Fluid-Vorratsbehälters (6) in der Halterung so, dass ein Benutzer den Fluidstand im Vorratsbehälter (6) ermitteln kann;
Aktivieren der Mechanik, wobei das Aktivieren bewirkt, dass die perforierte Ausgabeplatte (200) am Ausgabeende des Kapillarelement schwingt und Fluid von der Ausgabeplatte weg und in die Umluft pumpt; und
Erregen der Mechanik mittels der internen Stromquelle;
wobei das Verfahren weiterhin das länger andauernde Drücken eines Tasters auf dem Bedienfeld (8) als ein vorbestimmtes Zeitintervall beinhaltet, so dass die Mechanik Fluid stetig ausgibt, bis der Taster freigegeben wird.

14. Verfahren nach Anspruch 13, das weiterhin beinhaltet, den Fluidstand im auswechselbaren Fluid-Vorratsbehälter zu kontrollieren, um zu bestimmen, ob im Vorratsbehälter ein erster oder ein zweiter Füllstand herrscht.

## Revendications

1. Dispositif pour distribuer un fluide, comprenant :
un boîtier (4) ayant une source d'alimentation interne et un ensemble de montage (150) adapté pour recevoir un réservoir de fluide remplaçable (6) qui inclut un élément capillaire (218) pour un mouvement du fluide jusqu'à une extrémité de décharge de celui-ci ;
un mécanisme (124) à l'intérieur du boîtier (4) et alimenté par la source d'alimentation interne pour faire vibrer une plaque de décharge perforée (200) disposée au voisinage de l'extrémité de décharge de l'élément capillaire, le mécanisme (124) assurant un mouvement vibratoire suffisant dans un état de distribution pour pomper le fluide à partir de l'extrémité de décharge à travers la plaque de décharge (200) et dans l'atmosphère ; et
un panneau de commande (8) supporté par le boîtier (4) et disposé sous l'ensemble de montage (150), dans lequel le panneau de commande (8) fournit une interface à un utilisateur pour sélectionner au moins un parmi un mode de fonctionnement temporisé, un mode de fonctionnement automatique dépendant d'une sortie de capteur développée par un capteur, et un mode de fonctionnement manuel, et
dans lequel l'ensemble de montage (150) est en outre adapté pour recevoir le réservoir de fluide remplaçable (6) d'une manière qui permet à celui-ci d'être visuellement inspecté pendant un état utilisé ;
dans lequel l'interface comprend un bouton d'activation manuelle (30) pour activer le mécanisme, et
**caractérisé en ce que** ledit dispositif est conçu pour amener, lors d'un enfoncement du bouton d'activation, le mécanisme (124) à distribuer du fluide atomisé à partir du dispositif pendant une période de pulvérisation manuelle d'entre environ 1 seconde et environ 7 secondes, et un enfoncement continu du bouton d'activation manuelle (30) pendant une durée spécifiée amène le mécanisme à distribuer du fluide atomisé en continu jusqu'à ce que le bouton d'activation manuelle (30) soit relâché.

2. Dispositif selon la revendication 1, dans lequel le réservoir de fluide remplaçable (6) est reçu à l'intérieur de l'ensemble de montage (150).

3. Dispositif selon la revendication 2, dans lequel le réservoir de fluide remplaçable (6) donne une indication à l'utilisateur de si le réservoir de fluide (6) doit être remplacé.

4. Dispositif selon la revendication 1, dans lequel l'interface est positionnée pour permettre à l'utilisateur de sélectionner le au moins un parmi le mode de fonctionnement temporisé, le mode de fonctionnement automatique dépendant de la sortie de capteur, et le mode de fonctionnement manuel d'une manière qui n'entrave pas le fluide pompé pendant l'état de distribution.

5. Dispositif selon la revendication 1, dans lequel l'interface comprend un cadran qui peut tourner entre une position active et une position non active ; dans lequel la rotation du cadran jusqu'à une position particulière établit un intervalle de temps de repos pendant lequel le mécanisme reste inactif, et dans lequel l'activation du mécanisme a lieu à l'expiration de l'intervalle de temps de repos.

6. Dispositif selon la revendication 5, dans lequel l'intervalle de temps de repos est entre environ 0 seconde et un état de pulvérisation inactif en continu.

7. Dispositif selon la revendication 5, dans lequel le mécanisme peut être actionné pendant l'intervalle de temps de repos par un signal d'activation.

8. Dispositif selon la revendication 7, dans lequel le signal d'activation est développé sur la sortie de capteur ou est développé par l'activation manuelle d'un commutateur.

9. Dispositif selon la revendication 1, dans lequel le capteur comprend au moins un parmi un capteur de mouvement et un capteur de détection de composé malodorant.

10. Dispositif selon la revendication 1, dans lequel l'activation du mécanisme amène le fluide à être émis pendant une période de pulvérisation particulière.

11. Dispositif selon la revendication 10, dans lequel la période de pulvérisation a une durée entre environ une seconde et entre environ sept secondes.

12. Dispositif selon la revendication 1, dans lequel le mécanisme (124) pour faire vibrer une plaque de décharge perforée (200) est un élément piézoélectrique.

13. Procédé pour distribuer un fluide à partir d'un distributeur selon la revendication 1, comprenant :
fournir un boîtier (4) ayant une source d'alimentation interne, un ensemble de montage (150) pour recevoir un réservoir de fluide remplaçable (6), un mécanisme (124) pour faire vibrer une plaque de décharge perforée (200), et un panneau de commande (8) pour activer le distributeur ; dans lequel le mécanisme peut être activé par au moins un parmi un mode de fonctionnement temporisé, un mode de fonctionnement automatique dépendant d'une sortie d'un capteur, et un mode de fonctionnement manuel,
maintenir le réservoir de fluide remplaçable (6) à l'intérieur de l'ensemble de montage (150), le réservoir de fluide (6) incluant un élément capillaire pour le mouvement d'un fluide jusqu'à une extrémité de décharge de celui-ci ;
fournir un fluide pour une distribution à l'intérieur du réservoir de fluide remplaçable (6) ;
orienter le réservoir de fluide remplaçable (6) à l'intérieur de l'ensemble de montage (150) pour permettre à un utilisateur de déterminer un niveau du fluide à l'intérieur du réservoir de fluide (6) ;
activer le mécanisme dans lequel l'activation amène la plaque de décharge perforée (200) à vibrer au voisinage de l'extrémité de décharge de l'élément capillaire et pomper le fluide à partir de la plaque de décharge et dans l'atmosphère ; et
mettre sous tension le mécanisme par la source d'alimentation interne ;
le procédé comprenant en outre les étapes de presser un bouton sur le panneau de commande (8) plus longtemps qu'une période prédéterminée, ce qui amène le mécanisme à distribuer du fluide de manière continue jusqu'à ce que le bouton soit relâché.

14. Procédé selon la revendication 13, incluant en outre l'étape de surveiller le niveau du fluide à l'intérieur du réservoir de fluide remplaçable pour déterminer si le réservoir de fluide est dans un premier état plein ou dans un second état re-rempli.
